# EUROPEAN PATENT SPECIFICATION

(11) **EP 3 479 874 B1**
(45) Date of publication and mention of the grant of the patent: **30.12.2020**
(21) Application number: 17820443.4
(22) Date of filing: 19.06.2017
(51) Int. Cl.: A61N 5/10

(54) **BODY INSERTABLE DEVICE HAVING ADJUSTABLE RADIATION EMISSION DIRECTION**
IN DEN KÖRPER EINFÜHRBARE VORRICHTUNG MIT EINSTELLBARER STRAHLUNG EMISSIONSRICHTUNG
DISPOSITIF INSÉRABLE DANS LE CORPS AYANT UNE DIRECTION D'ÉMISSION DE RAYONNEMENT RÉGLABLE

(30) Priority: 01.07.2016 KR 20160083434
(43) Date of publication of application: 08.05.2019
(73) Proprietor: National Cancer Center, Goyang-si, Gyeonggi-do 10408 (KR); Korea Institute of Radiological & Medical Sciences, Seoul 01812 (KR); The Asan Foundation, Seoul 05505 (KR); University Of Ulsan Foundation For Industry Cooperation, Ulsan 44610 (KR)
(72) Inventor: LIM, Young Kyung, Paju-si Gyeonggi-do 10907 (KR); KWAK, Jung Won, Namyangju-si Gyeonggi-do 12208 (KR); KIM, Dae yong, Goyang-si Gyeonggi-do 10360 (KR); KIM, Yeon Joo, Goyang-si Gyeonggi-do 10417 (KR); KIM, Joo Young, Goyang-si Gyeonggi-do 10322 (KR); MIN, Soon Ki, Goyang-si Gyeonggi-do 10415 (KR); JUNG, Young Dae, Seoul 06074 (KR); JEONG, Ho Jin, Jinju-si Gyeongsangnam-do 52629 (KR); CHOI, Sang Hyoun, Seoul 07949 (KR); HWANG, Ui Jung, Goyang-si Gyeonggi-do 10374 (KR)
(74) Representative: Habermann, Hruschka & Schnabel
(86) International application number: PCT/KR2017/006381
(87) International publication number: WO 2018/004166

(56) References cited:
- EP-A1- 2 239 005
- WO-A1-2014/021947
- JP-B2- 4 602 356
- KR-A- 20090 074 145
- KR-Y1- 200 394 440
- US-A- 3 060 924
- US-A1- 2003 149 327
- US-A1- 2009 209 805

## Description

### Technical Field

The present invention relates to an insertable device and, more particularly, to a body insertable device for brachytherapy.

### Background Art

Radiation therapy for cancer patients may be classified largely into two types: external beam radiation therapy where radiation is delivered to a tumor from a radiation source placed outside a patient's body, and brachytherapy where radiation is delivered from a radiation source placed inside a patient's body.

Among them, the brachytherapy is carried out by inserting the radiation source into a tumor and delivering a high radiation dose to the tumor, and there is an advantage that the radiation dose transmitted to surrounding healthy organs may be reduced significantly. Devices allowing insertion of radiation sources are described e.g. in publications US 3060924 and EP 2239005.

In the brachytherapy, radiation is emitted by moving the radiation source through an insertable device inserted into a human body, but there is a problem that a technology for adjusting three-dimensional radiation intensity using the insertable device by adjusting a radiation direction has not been developed enough.

In particular, when a bent insertable device needs to be used to emit radiation to a tumor which is located like uterine cancer, it is difficult to assign an intensity adjusting function to the insertable device.

### Disclosure

### Technical Problem

An object of the present invention is to provide an insertable device for brachytherapy, the device which is bent and capable of adjusting radiation intensity.

### Technical Solution

The above objects of the present invention can be achieved by an insertable device including: an outer body including a first outer body extending in a long manner, a second outer body extending in a long manner, and a bent part for connecting the first outer body and the second outer body such that the first outer body and the second outer body are bent; a first inner body positioned inside the first outer body, and having an accommodation space in which a radiation source is accommodated; a second inner body positioned inside the second outer body, having a guide space enabling the radiation source to pass therethrough, and being capable of operating the rotation thereof; and an inner connection part positioned in correspondence to the bent part inside the outer body, having a connection space communicating with a guide space and the accommodation space such that the radiation source is allowed to pass therethrough, and connecting the first inner body and the second inner body, wherein the first inner body is separated from the first outer body and rotated by the rotation of the second inner body.

The accommodation space may be formed to extending in a long manner along the first inner body.

The accommodation space may be formed in a trench form within the first inner body.

The insertable device may further include a position limiting part positioned close to the first outer body within the accommodation space and limiting a position of the radiation source.

A vertical cross section of the first inner body in a lengthwise direction may be in a circular shape, and the accommodation space may accommodate the radiation source at an eccentric position.

The inner connection part may include a rotation power mechanism.

The rotation power mechanism may include at least one of a flexible shaft, a bellows, or a joint.

One end of the inner connection part may be fixed to the first inner body, and the other end of the inner connection part may be connected to the second inner body.

The inner connection part and the outer body may be spaced from each other, such that the inner connection part rotates in correspondence to the outer body when the inner connection part is transformed in response to rotation of the second inner part.

The accommodation space may include: a first accommodation space; and a second accommodation space positioned between the first accommodation space and the connection space, and the second accommodation space may have a cross-sectional area greater than a cross-sectional area of the first accommodation space.

The cross-sectional area of the second accommodation space may increase in proportion to proximity to the connection space.

The guide space may include: a first guide space; and a second guide space positioned between the first guide space and the connection space, and the second guide space may have a cross-sectional area greater than a cross-sectional area of the first guide space.

The cross-sectional area of the second guide space may increase in proportion to proximity to the connection space.

The first inner body may be made of a material with excellent radiation shielding performance, compared to the first outer body.

An angle of emission of radiation from the radiation source to an outside of the first outer body may be 5 degrees to 180 degrees.

### Advantageous Effects

According to the present invention, there is provided an insertable device for brachytherapy, which is bent and capable of adjusting radiation intensity.

### Description of Drawings

FIG. 1 is a perspective view of an insertable device according to an embodiment of the present invention.
FIG. 2 is an exploded perspective view of an insertable device according to an embodiment of the present invention.
FIG. 3 illustrates inner configurations of a portion A of FIG. 1.
FIGS. 4A and 4B illustrate a change in a position of an accommodation space in which a radiation source is accommodated in an insertable device according to an embodiment of the present invention.
FIG. 5 illustrates a cross-sectional view taken along line V-V' of FIG. 1.
FIGS. 6 and 7 illustrate adjustment of a radiation direction in an insertable device according to the present invention.
FIGS. 8 and 9 are for explanation of cancer treatment using an insertable device according to the present invention.
FIG. 10 illustrates an exemplary variation of an insertable device according to the present invention.

### Mode for Invention

Hereinafter, the present invention will be described in detail with reference to the accompanying drawings.

The accompanying drawings are only examples shown in order to describe the technical scope of the present invention in more detail. Therefore, the technical scope of the present invention is not limited to shapes of the accompanying drawings.

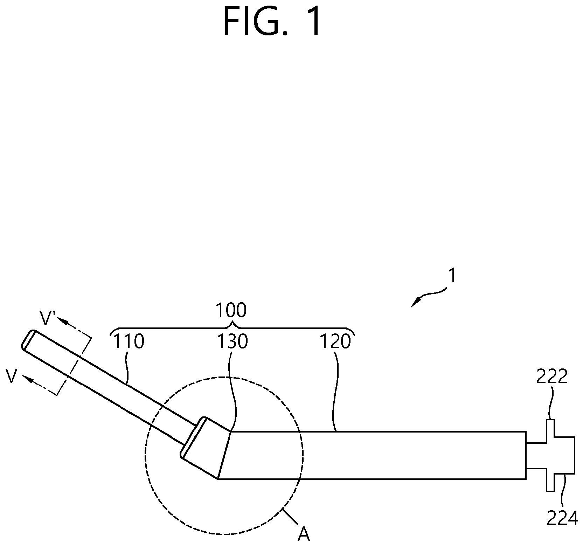

An insertable device according to an embodiment of the present invention is described with reference to FIGS. 1 to 5.

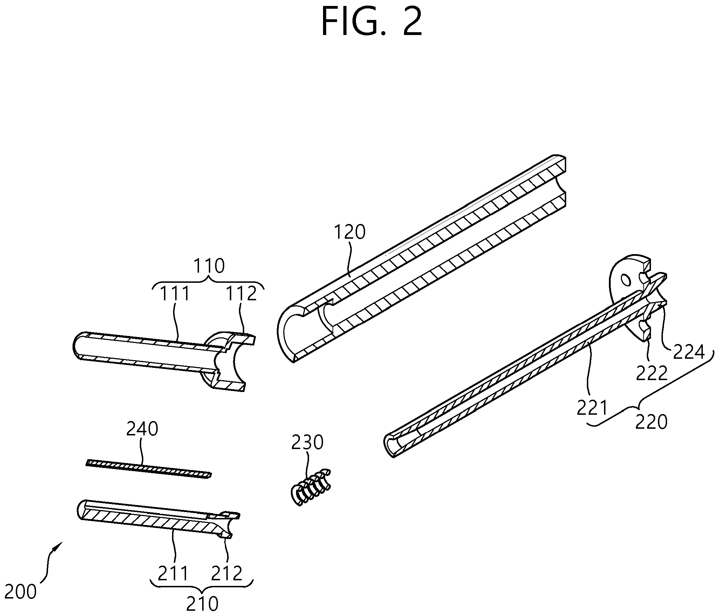

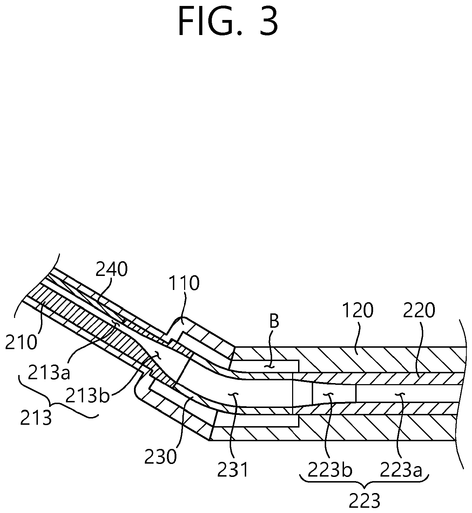

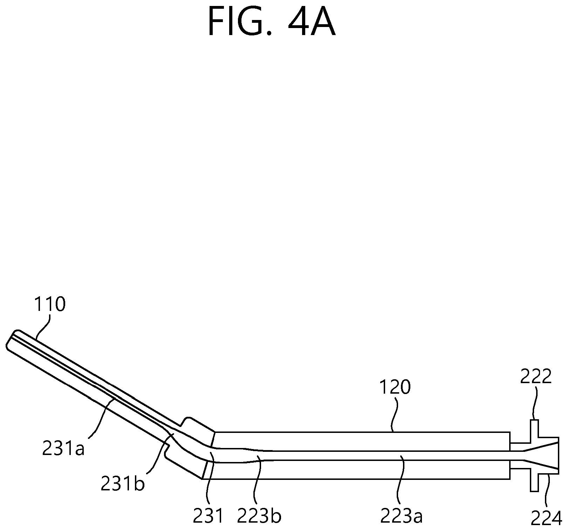

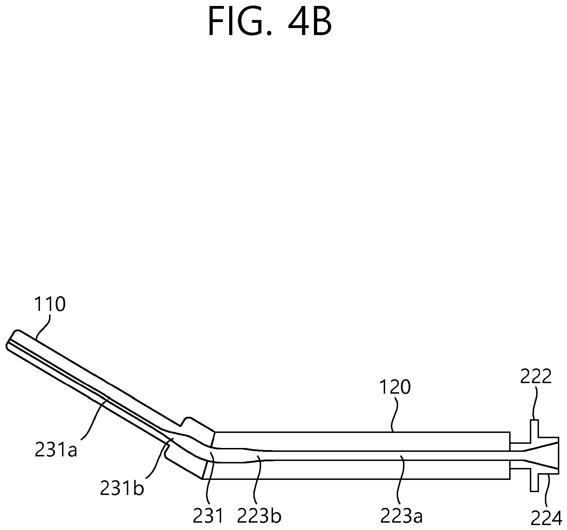

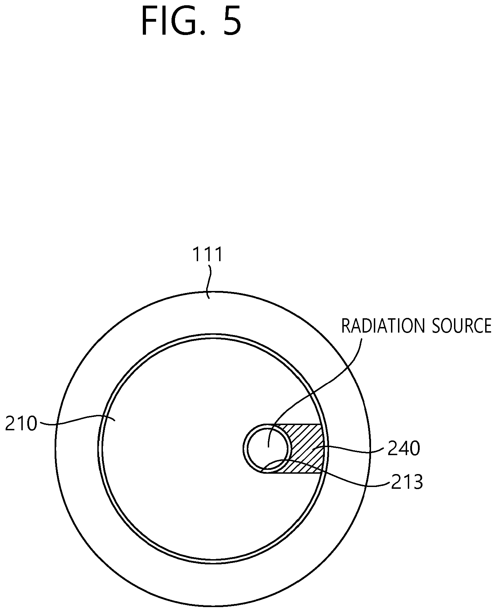

FIG. 1 is a perspective view of an insertable device according to an embodiment of the present invention, FIG. 2 is an exploded perspective view of an insertable device according to an embodiment of the present invention, FIG. 3 illustrates inner configurations of a portion A of FIG. 1, FIGS. 4A and 4B illustrate a change in a position of an accommodation space in which a radiation source is accommodated in an insertable device according to an embodiment of the present invention, FIG. 5 illustrates a cross-sectional view taken along line V-V' of FIG. 1. FIG. 2 illustrates a perspective view of a cross section in a lengthwise direction.

An insertable device 1 according to an embodiment of the present invention is largely comprised of an outer body 100 and an inner body 200.

The outer body 100 defines an overall outer appearance of the insertable device 1, and accommodates the inner body 200 in an empty space therein. In this embodiment, an operator 222 in the inner body 200 is exposed to an outside of the outer body 100.

The outer body 100 is in an overall thin and long shape and bent. Specifically, the outer body 100 includes a first outer body 110, a second outer body 120, and a bent part 130, and the first outer body 110 and the second body 120 are connected by the bent part 130 such that the first outer body and the second outer body are bent.

Such an outer body 100 may be provided in various manners. The outer body 100 may be provided as an overall integrated body or may be provided as the first outer body 110 and the second outer body 120 are manufactured individually and coupled thereto by welding or the like. In addition, the outer body 100 may be in a form which is made such that the first outer body 110 and/or the second outer body 120 are manufactured as a plurality of parts and coupled to each other.

Each of the first outer body 110 and the second outer body 120 may have a vertical cross section in a lengthwise direction, which is in a circular shape, and a cross section of an inner space of each of the first outer body 110 and the second outer body 120 is also in a circular shape. According to an orientation of the lengthwise direction, a cross-sectional area of each of the first outer body 110 and the second outer body 120, and a cross-sectional area of the inner space of each of the first outer body 110 and the second outer body 120 change. Overally, a cross-sectional area of the second outer body 120 is greater than a cross-sectional area of the first outer body 110. In another embodiment, a cross section of the first outer body 110 and/or the second outer body 120 may be in an elliptical shape rather than the circular shape.

The first outer body 110 of the outer body 100 is primarily brought into contact or close to a treatment target (tumor or the like), and, while the first outer body 110 is brought into contact or close to the treatment target, radiation is emitted toward the treatment target from a radiation source placed inside the inner body 200. The first outer body 110 may be made of a metallic material with a low atomic number such as titanium or a non-metallic material such as plastic.

Part of the second outer body 120 is inserted into a human body, and the other part is exposed to an outside. The second outer body 120 may be made of a metallic material such as stainless steel or a non-metallic material such as plastic.

The inner body 200 includes a first inner body 210, a second inner body 220, an inner connection part 230, and a position limiting part 240.

The first inner body 210 includes a first part 211 extending in a long manner, and a second part 212 extending in width.

An accommodation space 213 extending in a long manner along a lengthwise direction is formed in the first inner body 210. The accommodation space 213 includes a first accommodation space 213a formed in the first part 211, and a second accommodation space 213b formed in the second part 212.

The first accommodation space 213a is formed in a trench form, and the second accommodation space 213b has a cross-sectional area expanded than a cross section of the first accommodation space 213a. Specifically, the second accommodation space 213b has a funnel shape, of which a cross-sectional area increases in proportion to proximity to the inner connection part 230.

The second inner body 220 is comprised of a first part 221 extending in a long manner, and a second part (an operator 222) extending in width. The operator 222 is exposed to an outside of an end of the outer body 100.

A guide space 223 extending in a long manner along a lengthwise direction is formed in the second inner body 220. The guide space 223 includes a first guide space 223a and a second guide space 223b, and the second guide space 223b is formed at a portion adjacent to the inner connection part 230 and has a cross-sectional area greater than a cross-sectional area of the first guide space 223a. Specifically, the second guide space 223b has a funnel shape, of which a cross-sectional area increases in proportion to proximity to the inner connection part 230.

Meanwhile, a third part 224 is provided on an outside of the second part 222. The third part 224 serves to connect an external radiation source to the guide space 223.

The inner connection part 230 is provided at a portion corresponding to a bent part 130 of the outer body 100, and has a connection space 231 therein. One end of the inner connection part 230 is coupled to the first inner body 210, and the other part of the inner connection part 230 is coupled to the second inner body 220 and bent in correspondence to the bent part 130. Connection of the inner connection part 230 and the inner body 210 and 220 may be performed by welding or brazing.

The inner connection part 230 is, but not limited to, a rotation power transmitting mechanism between two shafts that intersect each other and may be at least one of a flexible shaft, a bellows, or a joint. In particular, the inner connection part 230 may be a pipe-shaped flexible shaft and may be especially made of a high-carbon steel wire. In the present invention, the inner connection part 230 may have various shapes as long as the inner connection part 230 maintains an accommodation space therein and transmits rotating motion of the second inner body 220 to the first inner body 210 while corresponding to a shape of the outer body 100.

The position limiting part 240 is in a thin and long shape, and positioned at the periphery of the first accommodation space 213a. A radiation source is positioned in an empty space under the position limiting part 240 of the first accommodation space 213a (that is, a portion close to the center of the first inner body 210). That is, with the radiation source being inserted, the first outer body 110, the position limiting part 240, and the radiation source are arranged in order.

The second part (the operator 222) is exposed to an outside, and a user rotates the operator 222 to change an emission direction and adjust radiation intensity.

Insertion of the radiation source in the above-described insertable device 1 will be described.

The radiation source is connected to an end of a thin and long wire, which is provided close to an external brachytherapy device, and emits radiation.

The radiation source passes through the guide space 223 formed within the second inner body 220 and through the connection space 231 formed within the inner connection part 230, and is then finally positioned within the accommodation space 213 formed in the first inner body 210. A position of the radiation source in a lengthwise direction in the accommodation space 213 may be adjusted in various ways.

In a path of insertion of the radiation source, both ends of the connection space 231 with a diameter greater than a diameter of the first guide space 223a and a diameter of the first accommodation space 213a are connected to each other by the funnel-shaped second guide space 223b and the funnel-shaped second accommodation space 213b. Due to this structure, the guide space 223, the connection space 231, and the accommodation space 213 form a continuous structure without a step formed therein, so that the radiation source is prevented from being stopped by the inner body 200 when being inserted, and the insertion of the radiation source is stably guided.

In addition, the radiation source is prevented from being stopped by the inner body 200 during the process of recovering the radiation source, and removal of the radiation source may be stably guided.

In another embodiment, an additional guide pipe may be used for stable insertion and recovery of the radiation source. In this case, the guide pipe may be provided over the entire length of the inner body 200 or may be provided only in the connection space 231.

Hereinafter, adjustment of radiation intensity in response to adjustment of an emission direction of the inserted radiation source is described.

In FIG. 4A, the radiation source is inserted in an upward direction. In this state, if the operator 222 is rotated in the outside, the second inner body 220 is rotated with the outer body 100 being fixed. If the second inner body 220 is rotated, the inner connection part 230 connected thereto is transformed and thus rotated. In this case, an empty space B is formed between the inner connection part 230 and the outer body 100, as shown in FIG. 3, and thus, interference with the outer body 100 does not happen despite transformation of the inner connection part 230.

Meanwhile, in another embodiment, an inner connection part 300 is in contact with the outer body 100 or brought into contact with the outer body 100 when being rotated. In this case, the inner connector 300 may be rotated while being transformed in correspondence to an inner shape of the outer body 100.

If the inner connector part 230 is rotated, the first inner body 210 connected thereto is also rotated, and thus, the position of the accommodation space for the radiation source is changed to face a downward direction, as shown in FIG. 4B. In response to the change in the position of the radiation source, a radiation emission direction is changed.

Overally, the radiation emission direction is changed in response to rotation of the operator 222, and a degree of change in the radiation emission direction may be 10% to 100%, 30% to 100%, 50% to 100%, and 80% to 100% of a degree of rotation of the operator 222. In response to the rotation of the operator 222, the radiation emission direction may be rotated by the same angle of the rotation of the operator 222.

Configurations of the operator 222 may be changed in various ways. An additional device may be further included to reduce a degree of change in the radiation emission direction in response to a different degree of rotation. In another embodiment, the operator 222 may not be integrally formed with the second inner body 220, or may rotate the second inner body 220 through an additional configuration connected to the second inner body 220 without having the operator 222 in the second inner body 220. In yet another embodiment, the second inner body 220 may be rotated through an additional mechanical-electric device.

The emission direction of the radiation source is changed in the above process, and this will be described in more detail with reference to FIG. 5.

FIG. 5 illustrates the case where the radiation source is in the accommodation space 213 of the first inner body 210. The accommodation space 213 is in a trench shape and faces the first outer body 110.

The position limiting part 240 is positioned in an upper space of the accommodation space 213, that is, a space directly facing the first outer body 110. The position of the radiation source is limited to the inside of the accommodation space 213 by the position limiting part 240. Thus, the radiation source is positioned at a predetermined distance from an outer surface (a circumferential surface) of the first inner body 210, and the distance to the outer surface is maintained constantly by the position limiting part 240.

In this arrangement, the radiation source is not positioned at the center of the first inner body 210 but is positioned eccentrically from the center thereof. The first inner body 210 is made of a material with excellent radiation shielding performance compared to the first outer body 110, and, for example, the first inner body 210 may be made of tungsten or tungsten alloy. Since the first inner body 210 has a very small diameter, it is hard to completely shield radiation in an undesired direction. According to this embodiment, the radiation source is positioned eccentrically, and thus, the first inner body 210 in a direction (a direction C in FIG. 5) in which emission of radiation from the radiation source is not desired may have a relatively great thickness. Thus, it is possible to minimize emission of radiation in an undesired direction.

The position limiting part 240 may be made of a polymer material, and, for smooth rotation of the first inner body 210, the first inner body 210 and the first outer body 110 may be spaced apart from each other and a lubricating film may be formed therebetween.

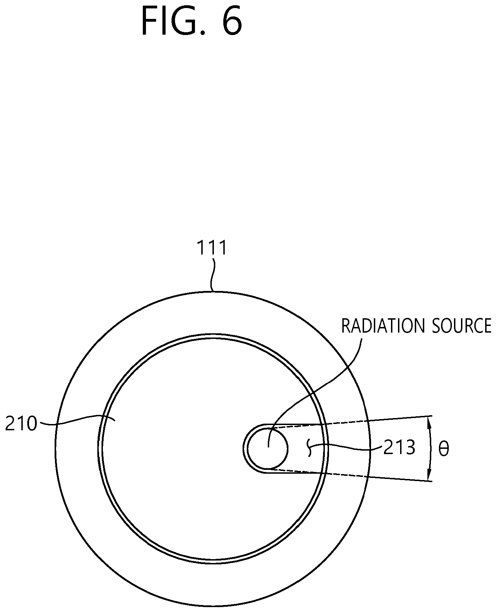

Using the insertable device according to the present invention, the brachytherapy may be performed effectively, and this is described with reference to FIGS. 6 to 9.

First, it is possible to effectively limit a radiation emission angle according to the present invention. Radiation is evenly emitted in a direction of 360 degrees from the radiation source. According to the present invention, as illustrated in FIG. 5, the radiation source is spaced from the circumferential surface of the first inner body 210. Thus, an emission angle (Θ in FIG. 6) of emission of radiation from the radiation source is limited by the first inner body 210. The emission angle is determined depending on a distance between the radiation source and the circumferential surface of the first inner body 210, a size of the accommodation space 213, and a shape of the accommodation space 213. The radiation emission angle may be 5 degrees to 180 degrees, 5 degrees to 120 degrees, 5 degrees to 60 degrees, 5 degrees to 30 degrees, 10 degrees to 120 degrees, 10 degrees to 60 degrees, 10 degrees to 30 degrees, 30 degrees to 120 degrees, 30 degrees to 90 degrees, or 30 degrees to 60 degrees.

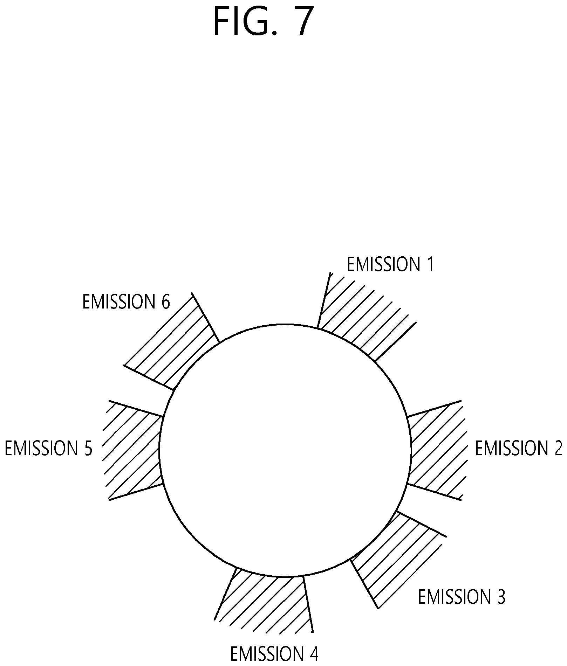

In addition, according to the present invention, the radiation direction may be adjusted continuously. That is, depending on a degree of rotation, the position of the radiation source may be changed, not in phases, but continuously. Thus, radiation may be emitted in a desired direction, as shown in FIG. 7.

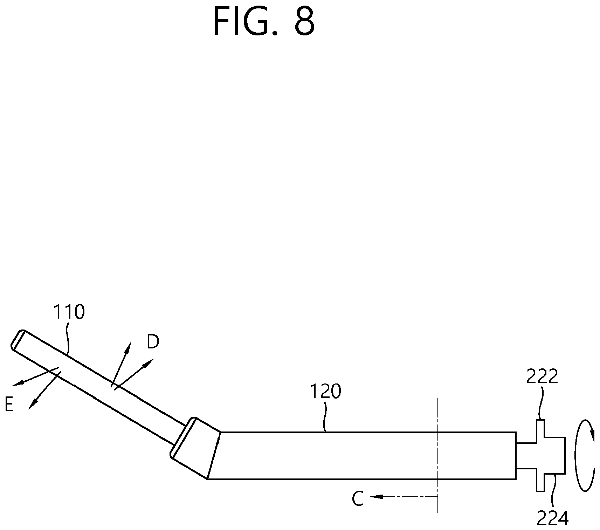

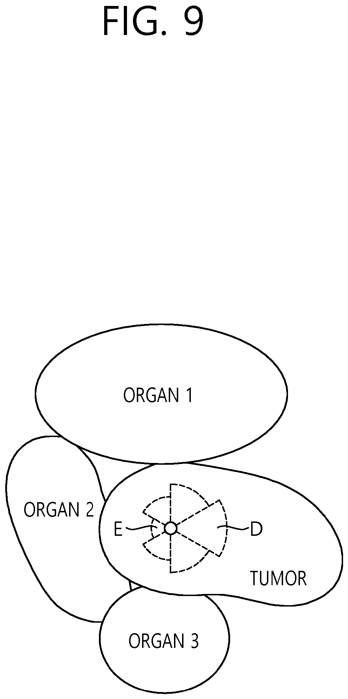

With reference to FIGS. 8 and 9, treatment of uterine cancer using the insertable device according to the present invention will be described.

The insertable device 1 is inserted into a human body and then the radiation source is introduced into the insertable device 1. When the insertion is completed, the right side at a position "C" in FIG. 8 is exposed to an outside of the human body. In addition, the first outer body 110 is inserted into a position of a tumor in accordance with a bent structure of the insertable device 1 inside the human body. In another embodiment, after the radiation source is introduced into the insertable device 1, the insertable device 1 may be inserted into the human body.

In this state, based on a shape and a position of a tumor found in a previous medical examination, the medical profession adjusts a position and a radiation direction of the radiation source according to a lengthwise direction of the first outer body 110. The radiation direction of the radiation source is adjusted by adjusting the operator, as described above.

When the position of the radiation source is determined with respect to a tumor shape as shown in FIG. 9, emission time is increased in a direction toward D where many tumors exist, so that sufficient radiation is emitted. On the other hand, emission time is reduced in a direction toward E where few tumors exist, so that emission of radiation toward surrounding organs is minimized.

During this treatment process, a radiation emission direction may be easily adjusted using the insertable device 1 of the present invention, and the radiation source may be easily inserted and removed.

In the above description, treatment of uterine cancer is exemplified, but the insertable device of the present invention is not limited thereto and is applicable even to head and neck cancer, esophageal cancer, rectal cancer, and the like.

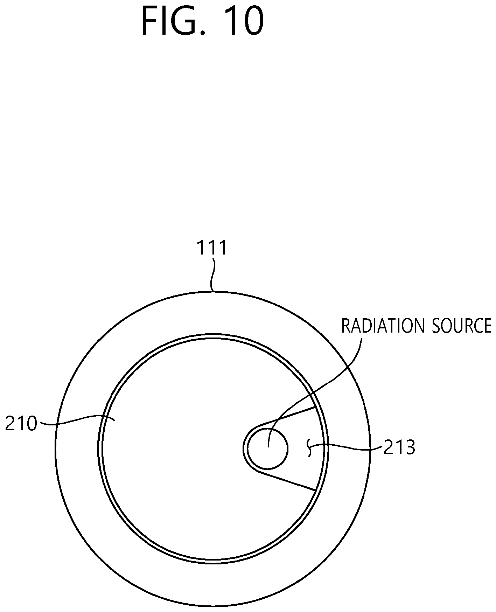

FIG. 10 illustrates a variation of an insertable device according to the present invention.

In an exemplary variation, the accommodation space 213 is formed to be widen in proportion to distance from the radiation source. Formation of the accommodation space 213 may be changed in various ways. Alternatively, the accommodation space 213 may be formed such that the radiation source is positioned at the center of the first inner body 210.

In another variation, an insertion angle, that is, an angle between the first outer body 110 and the second outer body 120, may be adjusted. The insertion angle may be adjusted and fixed outside a human body. Angle adjustment may be performed continuously or in stepwise, and a well-known configuration may be adapted for angle adjustment configuration.

In yet another variation, the inner connection part 230 is integrally formed with at least one of the first inner body 210 or the second inner body 220.

The above embodiments are illustrative purpose only for the present invention and an exemplary embodiment of the present invention not limited thereto. Since numerous variations and modifications will become apparent to those skilled in the art once the above disclosure is fully appreciated, the scope of the invention come within the scope of the appended claims.

## Claims

1. An insertable device (1) comprising:
an outer body (100) including a first outer body (110) extending in a long manner, a second outer body (120) extending in a long manner, and a bent part (130) for connecting the first outer body (110) and the second outer body (120) such that the first outer body (110) and the second outer body are (120) bent;
a first inner body (210) positioned inside the first outer body (110), and having an accommodation space (213) in which a radiation source is accommodated;
a second inner body (220) positioned inside the second outer body (120), having a guide space (223) enabling the radiation source to pass therethrough, and being capable of operating the rotation thereof; and
an inner connection part (230) positioned in correspondence to the bent part (130) inside the outer body (100), having a connection space (231) communicating with a guide space (223) and the accommodation space (213) such that the radiation source is allowed to pass therethrough, and connecting the first inner body (210) and the second inner body (220),
wherein the first inner body (210) is separated from the first outer body (110) and rotatable by the rotation of the second inner body (220).

2. The insertable device (1) of claim 1, wherein the accommodation space (213) is formed to extending in a long manner along the first inner body (210).

3. The insertable device (1) of claim 2, wherein the accommodation space (213) is formed in a trench form within the first inner body (210).

4. The insertable device (1) of claim 3, further comprising a position limiting part (240) positioned close to the first outer body (110) within the accommodation space (213) and limiting a position of the radiation source

5. The insertable device (1) of claim 2, wherein a vertical cross section of the first inner body (210) in a lengthwise direction is in a circular shape, and wherein the accommodation space (213) accommodates the radiation source at an eccentric position.

6. The insertable device (1) of claim 2, wherein the inner connection part (230) comprises a rotation power mechanism.

7. The insertable device (1) of claim 6, wherein the rotation power mechanism comprises at least one of a flexible shaft, a bellows, or a joint.

8. The insertable device (1) of claim 7, wherein one end of the inner connection part (230) is fixed to the first inner body (210), and the other end of the inner connection part (230) is connected to the second inner body (220).

9. The insertable device (1) of claim 7, wherein the inner connection part (230) and the outer body (100) are spaced from each other, such that the inner connection part (230) rotates in correspondence to the outer body (110) when the inner connection part (230) is transformed in response to rotation of the second inner part (220).

10. The insertable device (1) of claim 2,
wherein the accommodation space (213) comprises:
a first accommodation space (213a); and
a second accommodation space (213b) positioned between the first accommodation space (213a) and the connection space (231), and
wherein the second accommodation space (213b) has a cross-sectional area greater than a cross-sectional area of the first accommodation space (213a).

11. The insertable device (1) of claim 10, wherein the cross-sectional area of the second accommodation space (213b) increases in proportion to proximity to the connection space (231).

12. The insertable device (1) of claim 2,
wherein the guide space (223) comprises:
a first guide space (223a); and
a second guide space (223b) positioned between the first guide space (223a) and the connection space (231), and
wherein the second guide space (223b) has a cross-sectional area greater than a cross-sectional area of the first guide space (223a).

13. The insertable device (1) of claim 12, wherein the cross-sectional area of the second guide space (223b) increases in proportion to proximity to the connection space (231).

14. The insertable device (1) of claim 2, wherein the first inner body (210) is made of a material with excellent radiation shielding performance, compared to the first outer body (110).

15. The insertable device (1) of claim 2, wherein an angle of emission of radiation from the radiation source to an outside of the first outer body (110) is 5 degrees to 180 degrees.

## Patentansprüche

1. Einführbare Vorrichtung (1), aufweisend:
einen Außenkörper (100) mit einem ersten Außenkörper (110), der sich längs erstreckt, einem zweiten Außenkörper (120), der sich längs erstreckt, und einem gebogenen Teil (130) zum Verbinden des ersten Außenkörpers (110) und des zweiten Außenkörpers (120), so dass der erste Außenkörper (110) und der zweite Außenkörper (120) gebogen sind;
einen ersten Innenkörper (210), der im Inneren des ersten Außenkörpers (110) positioniert ist und einen Aufnahmeraum (213) aufweist, in dem eine Strahlungsquelle untergebracht ist;
einen zweiten Innenkörper (220), der im Inneren des zweiten Außenkörpers (120) positioniert ist und einen Führungsraum (223) aufweist, der es der Strahlungsquelle ermöglicht, durch diesen hindurchzugehen, und der in der Lage ist, deren Rotation zu betreiben; und
ein inneres Verbindungsteil (230), das in Entsprechung zu dem gebogenen Teil (130) im Inneren des Außenkörpers (100) positioniert ist, mit einem Verbindungsraum (231), der mit einem Führungsraum (223) und dem Aufnahmeraum (213) in Verbindung steht, so dass die Strahlungsquelle durch diesen hindurchtreten kann, und das den ersten Innenkörper (210) und den zweiten Innenkörper (220) verbindet,
wobei der erste Innenkörper (210) von dem ersten Außenkörper (110) getrennt und durch die Drehung des zweiten Innenkörpers (220) drehbar ist.

2. Einführbare Vorrichtung (1) nach Anspruch 1, bei der der Aufnahmeraum (213) so ausgebildet ist, dass er sich entlang dem ersten Innenkörper (210) erstreckt.

3. Einführbare Vorrichtung (1) nach Anspruch 2, bei der der Aufnahmeraum (213) innerhalb des ersten Innenkörpers (210) rinnenartig ausgebildet ist.

4. Einführbare Vorrichtung (1) nach Anspruch 3, die ferner ein Positionsbegrenzungsteil (240) aufweist, das nahe dem ersten Außenkörper (110) innerhalb des Aufnahmeraums (213) angeordnet ist und eine Position der Strahlungsquelle begrenzt.

5. Einführbare Vorrichtung (1) nach Anspruch 2, bei der ein vertikaler Querschnitt des ersten Innenkörpers (210) in einer Längsrichtung kreisförmig ist, und
bei der der Aufnahmeraum (213) die Strahlungsquelle in einer exzentrischen Position aufnimmt.

6. Einführbare Vorrichtung (1) nach Anspruch 2, bei der das innere Verbindungsteil (230) einen Drehkraftmechanismus aufweist.

7. Einführbare Vorrichtung (1) nach Anspruch 6, bei der der Drehkraftmechanismus mindestens eine flexible Welle, einen Balg oder ein Gelenk aufweist.

8. Einführbare Vorrichtung (1) nach Anspruch 7, bei der ein Ende des inneren Verbindungsteils (230) an dem ersten Innenkörper (210) befestigt ist und das andere Ende des inneren Verbindungsteils (230) mit dem zweiten Innenkörper (220) verbunden ist.

9. Einführbare Vorrichtung (1) nach Anspruch 7, bei der das innere Verbindungsteil (230) und der Außenkörper (100) voneinander beabstandet sind, so dass sich das innere Verbindungsteil (230) in Übereinstimmung mit dem Außenkörper (110) dreht, wenn das innere Verbindungsteil (230) als Reaktion auf die Drehung des zweiten Innenkörpers (220) umgeformt wird.

10. Einführbare Vorrichtung (1) nach Anspruch 2,
wobei der Aufnahmeraum (213) aufweist:
einen ersten Aufnahmeraum (213a); und
einen zweiten Aufnahmeraum (213b), der zwischen dem ersten Aufnahmeraum (213a) und dem Verbindungsraum (231) angeordnet ist, und
wobei der zweite Aufnahmeraum (213b) eine Querschnittsfläche aufweist, die größer ist als eine Querschnittsfläche des ersten Aufnahmeraums (213a).

11. Einführbare Vorrichtung (1) nach Anspruch 10, bei der die Querschnittsfläche des zweiten Aufnahmeraums (213b) proportional zur Nähe des Verbindungsraums (231) zunimmt.

12. Einführbare Vorrichtung (1) nach Anspruch 2,
wobei der Führungsraum (223) aufweist:
einen ersten Führungsraum (223a); und
einen zweiten Führungsraum (223b), der zwischen dem ersten Führungsraum (223a) und dem Verbindungsraum (231) angeordnet ist, und
wobei der zweite Führungsraum (223b) eine Querschnittsfläche aufweist, die größer ist als eine Querschnittsfläche des ersten Führungsraums (223a).

13. Einführbare Vorrichtung (1) nach Anspruch 12, bei der die Querschnittsfläche des zweiten Führungsraums (223b) proportional zur Nähe des Verbindungsraums (231) zunimmt.

14. Einführbare Vorrichtung (1) nach Anspruch 2, bei der der erste Innenkörper (210) aus einem Material mit ausgezeichneter Strahlungsabschirmungsleistung im Vergleich zum ersten Außenkörper (110) hergestellt ist.

15. Einführbare Vorrichtung (1) nach Anspruch 2, bei der ein Winkel der Strahlungsemission von der Strahlungsquelle zu einer Außenseite des ersten Außenkörpers (110) 5 Grad bis 180 Grad beträgt.

## Revendications

1. Dispositif insérable (1) comprenant :
un corps externe (100) comprenant un premier corps externe (110) s'étendant en longueur, un second corps externe (120) s'étendant en longueur, et une partie courbée (130) pour relier le premier corps externe (110) et le second corps externe (120) de telle sorte que le premier corps externe (110) et le second corps externe (120) sont pliés ;
un premier corps interne (210) positionné à l'intérieur du premier corps externe (110), et ayant un espace de réception (213) dans lequel une source de rayonnement est reçue ;
un second corps interne (220) positionné à l'intérieur du second corps externe (120), ayant un espace de guidage (223) permettant à la source de rayonnement de passer à travers celui-ci, et étant capable de mettre en œuvre la rotation de celui-ci ; et
une partie de connexion interne (230) positionnée en correspondance avec la partie pliée (130) à l'intérieur du corps externe (100), ayant un espace de connexion (231) communiquant avec un espace de guidage (223) et l'espace de réception (213) de telle sorte que la source de rayonnement peut passer à travers celui-ci, et reliant le premier corps interne (210) et le second corps interne (220),
dans lequel le premier corps interne (210) est séparé du premier corps externe (110) et peut tourner par l'intermédiaire de la rotation du second corps interne (220).

2. Dispositif insérable (1) selon la revendication 1, dans lequel l'espace de réception (213) est formé pour s'étendre en longueur le long du premier corps interne (210).

3. Dispositif insérable (1) selon la revendication 2, dans lequel l'espace de réception (213) est formé sous la forme d'une tranchée à l'intérieur du premier corps interne (210).

4. Dispositif insérable (1) selon la revendication 3, comprenant en outre une partie de limitation de position (240) positionnée à proximité du premier corps externe (110) à l'intérieur de l'espace de réception (213) et limitant une position de la source de rayonnement.

5. Dispositif insérable (1) selon la revendication 2, dans lequel une section transversale verticale du premier corps interne (210) dans une direction longitudinale est de forme circulaire, et
dans lequel l'espace de réception (213) reçoit la source de rayonnement dans une position excentrique.

6. Dispositif insérable (1) selon la revendication 2, dans lequel la partie de connexion interne (230) comprend un mécanisme de puissance de rotation.

7. Dispositif insérable (1) selon la revendication 6, dans lequel le mécanisme de puissance de rotation comprend au moins un parmi un arbre flexible, un soufflet, ou un joint.

8. Dispositif insérable (1) selon la revendication 7, dans lequel une extrémité de la partie de connexion interne (230) est fixée au premier corps interne (210), et l'autre extrémité de la partie de connexion interne (230) est reliée au second corps interne (220).

9. Dispositif insérable (1) selon la revendication 7, dans lequel la partie de connexion interne (230) et le corps externe (100) sont espacés l'un de l'autre, de telle sorte que la partie de connexion interne (230) tourne en correspondance avec le corps externe (110) lorsque la partie de connexion interne (230) est transformée en réponse à une rotation de la seconde partie interne (220).

10. Dispositif insérable (1) selon la revendication 2, dans lequel l'espace de réception (213) comprend :
un premier espace de réception (213a) ; et
un second espace de réception (213b) positionné entre le premier espace de réception (213a) et l'espace de connexion (231), et
dans lequel le second espace de réception (213b) a une aire de section transversale supérieure à une aire de section transversale du premier espace de réception (213a).

11. Dispositif insérable (1) selon la revendication 10, dans lequel la surface de section transversale du second espace de réception (213b) augmente proportionnellement à la proximité de l'espace de connexion (231).

12. Dispositif insérable (1) selon la revendication 2, dans lequel l'espace de guidage (223) comprend :
un premier espace de guidage (223a) ; et
un second espace de guidage (223b) positionné entre le premier espace de guidage (223a) et l'espace de connexion (231), et
dans lequel le second espace de guidage (223b) a une aire de section transversale supérieure à une aire de section transversale du premier espace de guidage (223a).

13. Dispositif insérable (1) selon la revendication 12, dans lequel l'aire de section transversale du second espace de guidage (223b) augmente proportionnellement à la proximité de l'espace de connexion (231).

14. Dispositif insérable (1) selon la revendication 2, dans lequel le premier corps interne (210) est fait d'un matériau avec une excellente performance de blindage anti-rayonnement, par comparaison au premier corps externe (110).

15. Dispositif insérable (1) selon la revendication 2, dans lequel un angle d'émission de rayonnement depuis la source de rayonnement vers l'extérieur du premier corps externe (110) est de 5 degrés à 180 degrés.
